# FASCICULE DE BREVET EUROPEEN

(11) **EP 3 694 505 B1**
(45) Date de publication et mention de la délivrance du brevet: **04.10.2023**
(21) Numéro de dépôt: 18773776.2
(22) Date de dépôt: 26.09.2018
(51) Int. Cl.: A61K 31/185, A61K 31/198, A61K 31/4415, A61K 31/51, A61K 31/525, A61K 33/06, A61P 25/00, A61P 25/28

(54) **COMPOSÉ ET COMPOSITION POUR UTILISATION DANS LE TRAITEMENT PRÉVENTIF ET/OU CURATIF DES MALADIES DU SYSTÈME NERVEUX CENTRAL CARACTÉRISÉES PAR UN DÉCLIN DE LA PLASTICITÉ NEURONALE, EN PARTICULIER CARACTÉRISÉES PAR UN DÉCLIN DE LA PLASTICITÉ SYNAPTIQUE**
VERBINDUNG UND ZUSAMMENSETZUNG ZUR VERWENDUNG BEI DER PRÄVENTIVEN UND / ODER HEILENDEN BEHANDLUNG VON ERKRANKUNGEN DES ZENTRALEN NERVENSYSTEMS, GEKENNZEICHNET DURCH EINEN ABFALL DER NEURONALEN PLASTIZITÄT, INSBESONDERE GEKENNZEICHNET DURCH EINEN ABFALL DER SYNAPTISCHEN PLASTIZITÄT
COMPOUND AND COMPOSITION FOR USE IN THE PREVENTIVE AND/OR CURATIVE TREATMENT OF DISEASES OF THE CENTRAL NERVOUS SYSTEM CHARACTERISED BY A DECLINE IN NEURONAL PLASTICITY, IN PARTICULAR CHARACTERISED BY A DECLINE IN SYNAPTIC PLASTICITY

(30) Priorité: 13.10.2017 BE 201705731
(43) Date de publication de la demande: 19.08.2020
(73) Titulaire: Synapharm Industrial Synthesis, 4432 Alleur (BE)
(72) Inventeur: DANHIER, Philippe, 7382 Audregnies (BE); AZZAM, Pascale, 4000 Liège (BE)
(74) Mandataire: Gevers Patents
(86) Numéro de dépôt international: PCT/EP2018/076133
(87) Numéro de publication internationale: WO 2019/072568

(56) Documents cités:
- FR-A1- 2 384 751
- US-A1- 2008 248 100
- SUÁREZ LUZ M ET AL: "Taurine content in different brain structures during ageing: effect on hippocampal synaptic plasticity", AMINO ACIDS, SPRINGER VERLAG, AU, vol. 48, no. 5, 23 janvier 2016 (2016-01-23), pages 1199-1208, XP035889627, ISSN: 0939-4451, DOI: 10.1007/S00726-015-2155-2 [extrait le 2016-01-23]
- AZLIANA JUSNIDA AHMAD JAFRI ET AL: "Protective effect of magnesium acetyltaurate against NMDA-induced retinal damage involves restoration of minerals and trace elements homeostasis", JOURNAL OF TRACE ELEMENTS IN MEDICINE AND BIOLOGY, vol. 39, 1 janvier 2017 (2017-01-01), pages 147-154, XP055484958, US ISSN: 0946-672X, DOI: 10.1016/j.jtemb.2016.09.005
- LAMBUK LIDAWANI ET AL: "Neuroprotective Effect of Magnesium Acetyltaurate Against NMDA-Induced Excitotoxicity in Rat Retina", NEUROTOXICITY RESEARCH, HARWOOD ACADEMIC PUBLISHERS, LAUSANNE, CH, vol. 31, no. 1, 27 août 2016 (2016-08-27), pages 31-45, XP036124819, ISSN: 1029-8428, DOI: 10.1007/S12640-016-9658-9 [extrait le 2016-08-27]
- J Durlach et al.: "Mg Acetyltaurinate as a photic inhibitor in photosensitive magnesium depletion: a physiological pathway in headache with photophobia treatment", , 2013, XP055485521, Extrait de l'Internet: URL:https://pdfs.semanticscholar.org/bd78/ 25ebb71f41bf84e60f84e5af9c65e03146c8.pdf?_ ga=2.77921361.439198391.1529396376-1410810 065.1502265378 [extrait le 2018-06-19]
- BAC P ET AL: "AUDIOGENIC SEIZURES IN MAGNESIUM-DEFICIENT MICE: EFFECTS OF MAGNESIUM PYRROLIDONE-2-CARBOXYLATE, MAGNESIUM ACETYLTAURINATE, MAGNESIUM CHLORIDE AND VITAMIN B-6", MAGNESIUM RESE, JOHN LIBBEY, LONDON, GB, vol. 6, no. 1, 1 janvier 1993 (1993-01-01) , pages 11-19, XP009048510, ISSN: 0953-1424

## Description

La présente invention se rapporte à un composé et à une composition pour utilisation dans le traitement préventif et/ou curatif des maladies du système nerveux central caractérisées par un déclin de la plasticité neuronale, en particulier caractérisées par un déclin de la plasticité synaptique, par exemple dans le traitement des maladies du système nerveux central affectant les processus cognitifs tels que l'apprentissage, la perception, l'attention, le raisonnement, le langage et/ou la mémoire, dans le traitement des maladies du système nerveux central telles que les maladies neurodégénératives de type Parkinson, Alzheimer et/ou sclérose latérale amyotrophique, dans le traitement des maladies du système nerveux central responsables de troubles de l'humeur, d'hyperexcitabilité, d'hyperactivité et/ou de stress, dans le traitement d'affections neurologiques telle que l'épilepsie.

La « plasticité neuronale » est un terme général qui comprend les mécanismes physiologiques, biochimiques et anatomiques collectifs permettant le développement du système nerveux durant les périodes embryonnaire et postnatale et qui, chez l'animal adulte (dont l'être humain), sont à la base des mécanismes de régénération des neurones endommagés et de la capacité d'adaptation du système nerveux central lorsque certaines parties de celui-ci dégénèrent et ne peuvent pas se régénérer. Par conséquent, les processus suivants sont impliqués lorsqu'on parle de plasticité neuronale : dénervation, ré-innervation, synaptogenèse, répression synaptique, expansion synaptique, germination des axones, régénération neuronale, développement et organisation de voies neuronales et de circuits pour remplacer ceux endommagés.

Il est désormais acquis que la plasticité neuronale est importante, même chez l'adulte, pour la formation de nouvelles connexions comme pour celle de nouveaux neurones (neurogenèse) ou de cellules gliales. Cependant, le vieillissement et les facteurs de stress peuvent altérer cette plasticité et favoriser le développement d'une atrophie cérébrale. Des anomalies de plasticité neuronale pourraient être l'un des facteurs contribuant au développement des maladies neuropsychiatriques, et ce aux différents stades de la vie.

La « plasticité synaptique » est une des composantes (un des mécanismes) impliquées dans la plasticité neuronale, ce qui implique qu'une amélioration/restauration de la plasticité synaptique contribue in-fine à une amélioration/restauration de la plasticité neuronale. Plus particulièrement, la « plasticité synaptique » concerne la communication entre neurones et plus spécifiquement l'efficacité de la transmission synaptique en lien direct avec des changements morphologiques des épines dendritiques. Les épines dendritiques sont des bourgeonnements des dendrites formant les domaines post-synaptiques qui permettent la transduction des signaux provenant des neurones pré-synaptiques. Ces structures se caractérisent par leur nature très plastique, leur morphologie et leur nombre étant modulés par l'activité neuronale.

Il est reconnu que la « plasticité synaptique » dépend de processus moléculaires permettant le maintien dans les épines dendritiques d'une optimisation de l'efficacité de transmission synaptique, que l'on appelle aussi la potentialisation à long terme (PLT), ou d'une dépréciation de l'efficacité de transmission synaptique, que l'on appelle la dépression à long terme (DLT). Les voies de signalisation qui régissent la plasticité synaptique mobilisent un grand nombre de récepteurs dont les récepteurs au glutamate ionotropique NMDAR et AMPAR et les récepteurs métabotropiques mGluR ainsi que les KA apparentés au mGluR.

La modification à long terme de l'efficacité de la transmission synaptique est l'un des mécanismes de base de la mémoire et de l'apprentissage. L'activation synchrone des éléments pré- et post-synaptiques provoque une augmentation de l'efficacité de la transmission synaptique, ou « potentialisation à long terme » (PLT). D'un point de vue cellulaire ou moléculaire, la plasticité neuronale est rapide et étendue, avec l'induction d'une potentialisation à long terme entraînant des modifications morphologiques des épines dendritiques. Notons qu'on définit la PLT comme une augmentation durable de l'efficacité synaptique suite à l'application d'une stimulation tétanique brève des fibres afférentes à la structure enregistrée.

Les sujets ciblés par la présente invention sont les sujets souffrant de maladies caractérisées par un déclin de la plasticité neuronale, ce déclin pouvant être observé par exemple chez des sujets également affectés par des pathologies dégénératives du système nerveux central, des traumas et des ischémies cérébrales. Un déclin de la plasticité neuronale est notamment responsable de pertes de mémoire, de troubles de l'apprentissage et du développement de certaines incapacités comme l'autisme et la dyslexie.

En d'autres termes, la plasticité neuronale ou neuroplasticité décrit la capacité du cerveau à remodeler ses connexions, dont les connexions synaptiques, en fonction de l'environnement et des expériences vécues par l'individu. Dans la vie foetale, des connexions entre neurones se mettent déjà en place. Plus tard, après la naissance, certaines connexions sont conservées et d'autres disparaissent. La plasticité neuronale peut intervenir lors de processus cognitifs tels que l'apprentissage, la perception, l'attention, le raisonnement, le langage ou la mémoire ou suite à des troubles ou des maladies du système nerveux central tels que les maladies neurodégénératives, un traumatisme ou une ischémie cérébrale, ou encore suite à des troubles de la mémoire ou à des troubles de l'attention.

Une restauration de la plasticité neuronale a été observée lors de recherches qui ont prouvé qu'un entraînement du cerveau ciblé permet de restaurer l'activité cérébrale et le fonctionnement cognitif chez des personnes ayant subi des lésions au cerveau. Une étude plus approfondie du cerveau des patients a permis de constater qu'après un programme d'entraînement du cerveau adapté, la performance cognitive perdue s'est améliorée et les régions lésées du cerveau se sont réactivées, voire même, dans certains cas, régénérées.

Ce processus de plasticité neuronale est possible grâce à deux changements principaux, à savoir un changement morphologique et un changement des propriétés électrochimiques des membranes.

Premièrement, le changement morphologique se fait par la succession d'une série d'étapes clés : la dénervation, la ré-innervation, la synaptogenèse, la répression synaptique, l'expansion synaptique, la germination des axones, la régénération neuronale, le développement et l'organisation des voies et des circuits neuronaux pour remplacer les circuits non utilisés ou endommagés.

Deuxièmement, le changement des propriétés électrochimiques de la membrane est observé par le changement des potentiels d'action le long de l'axone ou des potentiels post-synaptiques le long des dendrites. La propagation des signaux nerveux dépend de la composition de la membrane plasmique en canaux ioniques. Un changement de cette composition, qu'il soit qualitatif ou quantitatif, impactera la manière avec laquelle le signal électrique se propage. Le changement des propriétés électrochimiques de membrane peut être à court terme quand seul l'état des canaux ioniques est transitoirement modifié, ou à long terme, en général quand les canaux ioniques sont remplacés par d'autres. Par exemple, après l'acquisition de nouvelles connaissances, la communication ou la transmission synaptique entre les neurones impliqués est renforcée. Une meilleure communication entre les neurones facilite le déplacement des signaux électriques lors de leur parcours.

Comme expliqué plus haut, il y a un réel besoin de stimuler ou de restaurer la plasticité neuronale suite à des troubles ou des maladies du système nerveux central tels que les maladies neurodégénératives, un traumatisme ou une ischémie cérébrale, ou encore suite à des troubles de la mémoire ou à des troubles de l'attention. En effet, il a été observé que, lors de tels troubles ou maladies, l'activation et/ou la restauration de la plasticité neuronale chez les sujets atteints avait un effet bénéfique sur l'amélioration de leur état de santé en limitant un déclin des fonctions cérébrales.

Des méthodes non invasives (non médicamenteuses) ont été élaborées et sont prouvées comme ayant un rôle d'activation et/ou d'amélioration de la plasticité neuronale. En effet, afin d'améliorer la mémoire ou le processus d'apprentissage, il a été démontré que le sujet devait suivre cinq règles majeures : (1) éviter le stress et favoriser le sommeil, (2) exercer son cerveau, (3) se nourrir intelligemment, (4) développer un esprit social et (5) faire du sport. Même s'il est vrai qu'un effet bénéfique sur la plasticité neuronale a été observé chez les sujets suivant ces cinq règles, celles-ci sont toutefois difficiles voire impossibles à mettre en place chez des sujets atteints d'un trouble ou d'une maladie du système nerveux central. Par ailleurs, dans de tels cas, la situation est telle qu'il ne s'agit pas uniquement de stimuler la plasticité neuronale pour améliorer quelque chose qui fonctionne déjà bien (mémoire ou apprentissage), mais au contraire, il s'agit de stimuler voire de restaurer la plasticité neuronale afin de créer et/ou de réorganiser des neurones qui ont subi un grave dommage. En effet, lors d'un traumatisme, d'une ischémie cérébrale ou d'une maladie neurodégénérative, ou encore lors de troubles de la mémoire ou de troubles de l'attention, des neurones sont endommagés, voire complètement détruits et la plasticité neuronale est donc sollicitée pour créer ou réparer ceux-ci.

Des traitements médicamenteux favorisant la plasticité neuronale sont également connus, notamment des documents EP0441106 et EP1981522. Toutefois, les traitements divulgués dans les documents EP0441106 et EP1981522 présentent certaines limites notamment à cause du risque d'apparition d'effets secondaires non négligeables et de perturbations de voies de signalisation cruciales pour le bon fonctionnement du corps humain. Par ailleurs, de tels traitements médicamenteux représentent un coût important qui est difficilement pris en charge par le patient et les assurances maladies.

Le document US2008/248100 mentionne quant à lui, parmi une longue liste de composés, l'utilisation du taurate de magnésium (C₄H₁₂MgN₂O₆S₂) pour le traitement de maladies du système nerveux central caractérisées par un déclin de la plasticité neuronale. Toutefois, si le traitement envisagé selon le document US2008/248100 minimise les risques d'effets secondaires et s'avère être moins coûteux, son efficacité reste limitée. En effet, en pratique, il est observé que le taurate de magnésium est peu biodisponible, qu'il présente une faible bioéquivalence et que sa pénétration au sein des cellules est faible.

A ce jour, il n'existe donc malheureusement pas de traitement proposant un composé ou une composition dont le coût est acceptable, ce composé ou cette composition étant efficace et sûr(e) pour stimuler et/ou restaurer la plasticité neuronale tout en présentant une biodisponibilité et/ou une bioéquivalence et/ou une pénétration cellulaire adéquate pour assurer un traitement adéquat et optimisé des maladies caractérisées par un déclin de la plasticité neuronale.

Il existe donc un réel besoin de mettre au point et de formuler un composé et/ou une composition pour assurer un traitement adéquat et optimisé des maladies du système nerveux central caractérisées par un déclin de la plasticité neuronale, par activation et/ou restauration de la plasticité neuronale, ce composé et/ou cette composition présentant un coût acceptable, étant à la fois efficace et sans risque (sans effets secondaires néfastes) pour le corps humain et surtout présentant une biodisponibilité et/ou une bioéquivalence et/ou une pénétration cellulaire adéquate et optimisée.

Pour résoudre ce problème, il est prévu suivant l'invention, un composé et une composition pour utilisation dans le traitement préventif et/ou curatif des maladies du système nerveux central caractérisées par un déclin de la plasticité neuronale, en particulier caractérisées par un déclin de la plasticité synaptique, par exemple dans le traitement des maladies du système nerveux central affectant les processus cognitifs tels que l'apprentissage, la perception, l'attention, le raisonnement, le langage et/ou la mémoire, dans le traitement des maladies du système nerveux central telles que les maladies neurodégénératives de type Parkinson, Alzheimer et/ou sclérose latérale amyotrophique, dans le traitement des maladies du système nerveux central responsables de troubles de l'humeur, d'hyperexcitabilité, d'hyperactivité et/ou de stress, dans le traitement d'affections neurologiques telle que l'épilepsie, ledit composé étant du N-acétyl-taurinate de magnésium dihydraté comprenant deux molécules d'eau intrinsèques.

Dans le cadre de la présente invention, il a été déterminé, de façon surprenante, que le composé selon l'invention, c'est-à-dire le N-acétyl-taurinate de magnésium dihydraté comprenant deux molécules d'eau intrinsèques, présente une biodisponibilité, une bioéquivalence et une capacité de pénétration cellulaire significativement supérieures par rapport aux compositions de l'état de la technique. Par ailleurs, une composition selon l'invention présente un coût acceptable et est à la fois efficace et sûre pour le traitement de maladies du système nerveux central caractérisées par un déclin de la plasticité neuronale, en stimulant et/ou en restaurant la plasticité neuronale, par exemple dans le traitement des maladies du système nerveux central affectant les processus cognitifs tels que l'apprentissage, la perception, l'attention, le raisonnement, le langage et/ou la mémoire, dans le traitement des maladies du système nerveux central telles que les maladies neurodégénératives de type Parkinson, Alzheimer et/ou sclérose latérale amyotrophique, dans le traitement des maladies du système nerveux central responsables de troubles de l'humeur, d'hyperexcitabilité, d'hyperactivité et/ou de stress, dans le traitement d'affections neurologiques telle que l'épilepsie.

Un composé selon l'invention démontre un effet supérieur sur la stimulation et/ou la restauration de la plasticité neuronale pour le traitement d'une maladie ou d'un trouble du système nerveux central chez un sujet et ce grâce à sa double action. Un tel composé selon l'invention agit tout d'abord à un stade très précoce de la réparation neuronale (naissance d'un nouveau neurone) via l'activation du récepteur NMDAR et, dans un deuxième temps, agit sur la production du facteur BDNF (brain-derived nerotrophic factor) qui influence la plasticité synaptique dans le cerveau adulte.

Comme indiqué plus haut, il a été démontré qu'un composé selon l'invention, c'est-à-dire le N-acétyl-taurinate de magnésium dihydraté comprenant deux molécules d'eau intrinsèques, possède une efficacité supérieure en termes de biodisponibilité et de bioéquivalence. En effet, il a été mis en avant dans le cadre de la présente invention, que le composé selon l'invention présente une meilleure biodisponibilité grâce à l'action de la taurine acétylée sur la membrane cellulaire via son caractère lipophile.

Notons que le N-acétyl-taurinate de magnésium (C₈H₁₆MgN₂O₈S₂) est connu pour ses propriétés cyto-vasculo protectrices telles que des propriétés anti-aggrégantes plaquettaires et protectrices des thromboses veineuses et artérielles ainsi qu'une action stabilisatrice de la membrane érythrocytaire. En outre, un effet protectif du magnésium N-acétyl-taurinate contre le glaucome en agissant sur la dérégulation vasculaire a été démontré, tout comme les propriétés angio-protectrices du N-acétyl-taurinate de magnésium par son action anti-inflammatoire en restaurant les teneurs en eNOS (endothelial Nitric Oxide Synthase 3). Des études ont également démontré un effet bénéfique du magnésium N-acétyl-taurinate sur la neurotoxicité due à l'hyperexcitabilité des récepteurs glutamatergiques ionotropes. En effet, le magnésium N-acétyl-taurinate a une action intraneuronale non seulement sur le récepteur NMDA mais également sur les deux autres récepteurs ionotropes de l'acide glutamique à savoir les récepteurs AMPA et KA, ceux-ci étant impliqués dans la vitesse de la transmission synaptique. Il a été remarqué qu'un tel composé présente une analogie structurelle avec l'acide glutamique mais également l'acide kaïnique. Ainsi, le N-acétyl-taurinate de magnésium va cibler l'ensemble des récepteurs glutamatergiques NMDAR, AMPAR et KAR entraînant par conséquent l'inhibition des voies de signalisation en aval de ces récepteurs.

Par les termes « deux molécules d'eau intrinsèques », il est entendu, au sens de la présente invention, que les deux molécules d'eau sont inhérentes au N-acétyl-taurinate de magnésium dihydraté, c'est-à-dire qu'elles font partie intégrante du N-acétyl-taurinate de magnésium dihydraté, à la différence de l'eau d'hydratation qui pourrait être absorbée ou adsorbée par ce composé.

Le N-acétyl-taurinate de magnésium dihydraté (C₈H₂₀MgN₂O₁₀S₂), dénommé également magnésium N-acétyl-taurinate dihydraté, est un vecteur magnésique et un analogue magnésique de la taurine qui présente un poids moléculaire de 392,677 g/mol, deux molécules d'eau (H₂O) étant intrinsèques à la molécule de N-acétyl-taurinate de magnésium dihydraté.

En particulier, le N-acétyl-taurinate de magnésium dihydraté est celui qui est produit et commercialisé sous la marque ATAMg^{®} par la société Synapharm Industrial Synthesis.

Comme le N-acétyl-taurinate de magnésium non dihydraté, le N-acétyl-taurinate de magnésium dihydraté comprenant deux molécules d'eau intrinsèques possède différentes caractéristiques telles qu'un dérivé β aminé soufré, un acide sulfonique (non carboxylique), un N-acétylé, ne présente pas le caractère amphotère de la taurine (forme Zwitterion, c'est-à-dire qu'une charge positive et une charge négative se trouvent sur le même résidu) et optimise le caractère taurinergique intracellulaire. La charge électrique sur l'azote de la taurine étant supprimée par l'acétylation, seuls les électrons du cation Mg++ sont maintenus chélatés par les deux radicaux sulfoniques de la taurine. Cela conduit à un dérivé éthanamide (acétamide) au caractère lipophile plus marqué que celui de la taurine amphotère, ce qui facilite la pénétration au travers les phospholipides membranaires neuronaux. Les dérivés éthanamides (acétamides) caractérisent les molécules utilisées pour leurs actions nootropes (piracetam-like), anti-convulsivantes et anti-épileptiques (levitracetam-like).

Le poids moléculaire du N-acétyl-taurinate de magnésium dihydraté est de 392,677 g/mol et diffère de celui du N-acétyl-taurinate de magnésium non dihydraté (C₈H₁₆MgN₂O₈S₂) qui est de 356,656 g/mol. Cette forme qui n'est pas dihydratée est notamment décrite dans le document FR2384751 où le N-acétyl-taurinate de magnésium est obtenu par mélange de magnésie, de taurine, d'eau et d'acide acétique avant deux étapes successives de séchage, l'une sous vide à 100°C et l'autre avec un solvant de séchage, de telle sorte à obtenir des cristaux.

Lorsque le composé selon l'invention est le N-acétyl-taurinate de magnésium sous forme dihydratée comprenant deux molécules d'eau intrinsèques, il a été mis en évidence que la biodisponibilité, que la bioéquivalence et que la pénétration cellulaire par passage au travers des membranes sont encore améliorées et optimisées grâce à la présence des deux molécules d'eau intrinsèques au N-acétyl-taurinate de magnésium dihydraté, la présence de ces deux molécules d'eau permettant d'assurer une meilleure pénétration cellulaire du N-acétyl-taurinate de magnésium dihydraté.

Par ailleurs, il a été déterminé que le N-acétyl-taurinate de magnésium sous forme dihydratée comprenant deux molécules d'eau intrinsèques présente une stabilité au cours du temps qui est augmentée par rapport au N-acétyl-taurinate de magnésium non dihydraté, c'est-à-dire par rapport au N-acétyl-taurinate de magnésium anhydre. En d'autres termes, il a été mis en évidence que la forme dihydratée du N-acétyl-taurinate de magnésium conserve mieux ses propriétés au cours du temps en comparaison avec la forme anhydre du N-acétyl-taurinate de magnésium.

Il a en outre été déterminé que les deux molécules d'eau intrinsèques sont responsables de la formation d'un complexe stable par établissement de liaisons entre les deux molécules d'eau intrinsèques et le magnésium présents au sein du composé N-acétyl-taurinate de magnésium dihydraté. Plus particulièrement, le magnésium est lié à la N-acétyl-taurine et à l'eau par l'intermédiaire de liaisons non covalentes de type liaisons de coordination métal-ligand.

La formation d'un tel complexe est particulièrement avantageuse puisque ce dernier assure une stabilité accrue du composé : le magnésium reste fixé (piégé) au complexe et atteint ainsi d'autant mieux la zone ciblée, c'est-à-dire le cerveau afin d'assurer une restauration de la plasticité neuronale, en particulier une restauration de la plasticité synaptique, par exemple dans le traitement des maladies du système nerveux central affectant les processus cognitifs tels que l'apprentissage, la perception, l'attention, le raisonnement, le langage et/ou la mémoire, dans le traitement des maladies du système nerveux central telles que les maladies neurodégénératives de type Parkinson, Alzheimer et/ou sclérose latérale amyotrophique, dans le traitement des maladies du système nerveux central responsables de troubles de l'humeur, d'hyperexcitabilité, d'hyperactivité et/ou de stress, dans le traitement d'affections neurologiques telle que l'épilepsie.

Par le terme « biodisponibilité », il faut comprendre au sens de la présente invention, une propriété pharmacocinétique des médicaments, à savoir la fraction d'une dose qui atteint la circulation sanguine sous forme inchangée. La biodisponibilité est donc la mesure de la vitesse d'absorption et de la quantité de médicament absorbée. Ainsi, la composition selon l'invention permet d'obtenir un pourcentage de magnésium élevé dans le sang, ce qui implique une meilleure action du magnésium sur le métabolisme.

Comme indiqué plus haut, il a été mis en évidence une nette augmentation de la biodisponibilité et de la bioéquivalence du magnésium lorsque le composé selon l'invention est le N-acétyl-taurinate de magnésium dihydraté comprenant deux molécules d'eau intrinsèques. La pénétration cellulaire et le passage de la barrière hémato-encéphalique sont optimisés grâce à la présence des deux molécules d'eau intrinsèques au N-acétyl-taurinate de magnésium dihydraté, la présence de ces deux molécules d'eau permettant d'assurer une meilleure pénétration cellulaire du N-acétyl-taurinate de magnésium dihydraté.

Notons que la « bioéquivalence » reflète de manière plus réelle l'action métabolique et permet une comparaison entre des produits qui ont un même ingrédient actif (le magnésium par exemple).

Par ailleurs, comme indiqué plus haut, le composé selon l'invention est sûr et bénéfique (au-delà de ses propriétés exposées plus haut) pour le corps humain. En effet, le magnésium contribue au maintien de la balance des fluides et des électrolytes, à un métabolisme énergétique et une synthèse protéique normaux, à des fonctions musculaires adaptées, à la réduction de la fatigue et de l'asthénie, à maintenir une bonne dentition et une structure osseuse adéquate et contribue au fonctionnement normal du système nerveux et de la fonction psychique.

En outre, le N-acétyl-taurinate aide, quant à lui, la pénétration cellulaire du magnésium et de la taurine et agit sur le maintien de l'osmolarité cellulaire.

De préférence, le composé se présente sous une forme administrable oralement, par exemple sous forme d'un comprimé, d'une capsule, d'une gélule, d'un cachet, d'une poudre soluble, d'une solution huileuse, d'un comprimé effervescent ou d'une gélule molle.

Dans une forme particulière selon l'invention, le composé est administré à raison de 1 à 2 capsules par jour chez l'adulte, l'adolescent ou l'enfant.

D'autres formes de réalisation du composé suivant l'invention sont indiquées dans les revendications annexées.

La présente invention porte également sur une composition pour utilisation dans le traitement préventif et/ou curatif des maladies du système nerveux central caractérisées par un déclin de la plasticité neuronale, en particulier caractérisée par un déclin de la plasticité synaptique, par exemple dans le traitement des maladies du système nerveux central affectant les processus cognitifs tels que l'apprentissage, la perception, l'attention, le raisonnement, le langage et/ou la mémoire, dans le traitement des maladies du système nerveux central telles que les maladies neurodégénératives de type Parkinson, Alzheimer et/ou sclérose latérale amyotrophique, dans le traitement des maladies du système nerveux central responsables de troubles de l'humeur, d'hyperexcitabilité, d'hyperactivité et/ou de stress, dans le traitement d'affections neurologiques telle que l'épilepsie, ladite composition comprenant au moins du N-acétyl-taurinate de magnésium dihydraté comprenant deux molécules d'eau intrinsèques.

Par les termes « deux molécules d'eau intrinsèques », il est entendu, au sens de la présente invention, que les deux molécules d'eau sont inhérentes au N-acétyl-taurinate de magnésium dihydraté, c'est-à-dire qu'elles font partie intégrante du N-acétyl-taurinate de magnésium dihydraté, à la différence de l'eau d'hydratation qui pourrait être absorbée ou adsorbée par ce composé.

Le N-acétyl-taurinate de magnésium dihydraté (C₈H₂₀MgN₂O₁₀S₂), dénommé également magnésium N-acétyl-taurinate dihydraté, est un vecteur magnésique et un analogue magnésique de la taurine qui présente un poids moléculaire de 392,677 g/mol, deux molécules d'eau (H₂O) étant intrinsèques à la molécule de N-acétyl-taurinate de magnésium dihydraté.

En particulier, le N-acétyl-taurinate de magnésium dihydraté est celui qui est produit et commercialisé sous la marque ATAMg^{®} par la société Synapharm Industrial Synthesis.

Le poids moléculaire du N-acétyl-taurinate de magnésium dihydraté est de 392,677 g/mol et diffère de celui du N-acétyl-taurinate de magnésium non dihydraté (C₈H₁₆MgN₂O₈S₂) qui est de 356,656 g/mol. Cette forme qui n'est pas dihydratée est notamment décrite dans le document FR2384751 où le N-acétyl-taurinate de magnésium est obtenu par mélange de magnésie, de taurine, d'eau et d'acide acétique avant deux étapes successives de séchage, l'une sous vide à 100°C et l'autre avec un solvant de séchage, de telle sorte à obtenir des cristaux.

Lorsque, dans une composition selon l'invention, le N-acétyl-taurinate de magnésium est le N-acétyl-taurinate de magnésium dihydraté comprenant deux molécules d'eau intrinsèques, il a été mis en évidence que la biodisponibilité, que la bioéquivalence et que la pénétration cellulaire par passage au travers des membranes sont encore améliorées et optimisées grâce à la présence des deux molécules d'eau intrinsèques au N-acétyl-taurinate de magnésium dihydraté, la présence de ces deux molécules d'eau permettant d'assurer une meilleure pénétration cellulaire du N-acétyl-taurinate de magnésium dihydraté.

Il a en outre été déterminé que les deux molécules d'eau intrinsèques sont responsables de la formation d'un complexe stable par établissement de liaisons entre les deux molécules d'eau intrinsèques et le magnésium présents au sein du composé N-acétyl-taurinate de magnésium dihydraté. Plus particulièrement, le magnésium est lié à la N-acétyl-taurine et à l'eau par l'intermédiaire de liaisons non covalentes de type liaisons de coordination métal-ligand.

La formation d'un tel complexe est particulièrement avantageuse puisque ce dernier assure une stabilité accrue du composé : le magnésium reste fixé (piégé) au complexe et atteint ainsi d'autant mieux la zone ciblée, c'est-à-dire le cerveau afin d'assurer une restauration de la plasticité neuronale, en particulier une restauration de la plasticité synaptique, par exemple dans le traitement des maladies du système nerveux central affectant les processus cognitifs tels que l'apprentissage, la perception, l'attention, le raisonnement, le langage et/ou la mémoire, dans le traitement des maladies du système nerveux central telles que les maladies neurodégénératives de type Parkinson, Alzheimer et/ou sclérose latérale amyotrophique, dans le traitement des maladies du système nerveux central responsables de troubles de l'humeur, d'hyperexcitabilité, d'hyperactivité et/ou de stress, dans le traitement d'affections neurologiques telle que l'épilepsie.

Avantageusement, le N-acétyl-taurinate de magnésium dihydraté comprenant deux molécules d'eau intrinsèques est présent à raison de 40 à 60% en poids par rapport au poids total de la composition.

Dans une forme particulièrement avantageuse, la composition selon l'invention comprend en outre de la taurine à raison de 10 à 15% en poids par rapport au poids total de la composition.

Avantageusement, la composition selon l'invention comprend en outre au moins un composé magnésique additionnel choisi dans le groupe constitué des sels magnésiques d'acides aminés, du citrate de magnésium, du gluconate de magnésium, du lactate de magnésium, du malate de magnésium, du taurate de magnésium, du bisglycinate de magnésium, du glycérophosphate de magnésium, du thréonate de magnésium, leurs dérivés et leurs mélanges.

De préférence, selon l'invention, la composition comprend en outre du magnésium à raison de 8 à 15% en poids par rapport au poids total de la composition.

De plus, dans une forme de réalisation particulière, la composition comprend en outre au moins un acide aminé, par exemple de la thréonine.

De préférence, ledit au moins un acide aminé est la glycine présente à raison de 10 à 15% en poids par rapport au poids total de la composition.

De manière avantageuse, ledit au moins un acide aminé est la thréonine présente à raison de 10 à 15% en poids par rapport au poids total de la composition.

Dans une forme particulière, selon l'invention, la composition comprend en outre au moins une vitamine choisie dans le groupe constitué de la vitamine B1, B2, B6 et leurs mélanges.

Avantageusement, selon l'invention, ladite au moins une vitamine est la vitamine B1 présente à raison de 0,05 à 0,1% en poids par rapport au poids total de la composition.

De préférence, ladite au moins une vitamine est la vitamine B2 présente à raison de 0,05 à 0,1% en poids par rapport au poids total de la composition.

Dans une forme de réalisation particulièrement avantageuse, selon l'invention, ladite au moins une vitamine est la vitamine B6 présente à raison de 0,05 à 0,15 % en poids par rapport au poids total de la composition.

De façon avantageuse, la composition comprend en outre au moins un excipient pharmaceutiquement acceptable, de préférence quatre excipients pharmaceutiquement acceptables, choisi dans le groupe constitué du glycéryl béhénate, du stéarate de saccharose, de la cellulose microcristalline, du stéarate de magnésium, du dioxyde de silice, de la malodextrine de pois et leurs mélanges.

Dans une forme particulière, la composition selon l'invention se présente sous la forme d'un médicament.

De préférence, la composition selon l'invention se présente sous la forme d'un complément alimentaire.

Avantageusement, la composition selon l'invention se présente sous la forme d'une boisson.

Dans une forme de réalisation particulièrement avantageuse selon l'invention, la composition se présente sous une forme administrable oralement, par exemple sous forme d'un comprimé, d'une capsule, d'une gélule, d'un cachet, d'une poudre soluble, d'une solution huileuse, d'un comprimé effervescent ou d'une gélule molle.

De plus, dans une forme de réalisation particulière, la composition selon l'invention est administrée à raison de 1 à 2 capsules par jour chez l'adulte, l'adolescent ou l'enfant.

D'autres formes de réalisation de la composition suivant l'invention sont indiquées dans les revendications annexées.
La figure 1 illustre la potentialisation à long terme (%) mesurée sur des rats contrôles nourris normalement.
La figure 2 illustre la potentialisation à long terme (%) mesurée sur des rats carencés en magnésium par administration d'une nourriture pauvre en magnésium.
La figure 3 illustre la potentialisation à long terme (%) mesurée sur des rats carencés en magnésium mais supplémentés avec une composition selon l'invention comprenant du N-acétyl-taurinate de magnésium dihydraté comprenant deux molécules d'eau intrinsèques (ATAMg dihydraté) à raison de 50 mg/kg/jour de N-acétyl-taurinate de magnésium dihydraté comprenant deux molécules d'eau intrinsèques.

### Exemple

### 1. Effet d'une composition selon l'invention comprenant du N-acétyl-taurinate de magnésium dihydraté comprenant deux molécules d'eau intrinsèques sur la restauration de la plasticité synaptique

Des essais comparatifs ont été menés sur des rats afin d'y mesurer l'efficacité de la transmission synaptique et la plasticité synaptique, en particulier pour y mesurer une récupération de la plasticité synaptique.

Trois groupes de rats ont été constitué comme suit :
- Groupe 1 : rats contrôles nourris normalement ;
- Groupe 2 : rats carencés en magnésium par administration d'une nourriture pauvre en magnésium ;
- Groupe 3 : rats carencés en magnésium mais supplémentés avec une composition selon l'invention comprenant du N-acétyl-taurinate de magnésium dihydraté comprenant deux molécules d'eau intrinsèques (ATAMg dihydraté) à raison de 50 mg/kg/jour de N-acétyl-taurinate de magnésium dihydraté comprenant deux molécules d'eau intrinsèques (dans de l'eau). Cette supplémentation a débuté 40 jours après le début du régime carencé et a duré 24 jours.

Le tableau 1 ci-dessous reprend les quantités de magnésium (mg/kg/jour) ingérées par les rats de chacun des Groupes, dans la nourriture et dans l'eau qui leur est administrée.

**Tableau 1**

| | Nourriture | Eau | ATAMg dihydraté dans de l'eau | Quantité totale de Mg |
|---|---|---|---|---|
| Groupe 1 | 21 | 1,15 | 0 | 22,15 |
| Groupe 2 | 1,5 | 1,15 | 0 | 2,65 |
| Groupe 3 | 1,5 | 1,15 | 3,7 | 6,35 |

Les hippocampes des rats de chacun des groupes ont été disséqué afin de mesurer la plasticité synaptique. En particulier, la réponse synaptique a été mesurée entre les collatérales de Schaeffer et les dendrites de la région CA1. Une ligne de base a été enregistrée durant 30 minutes et correspond à la réponse synaptique de 40% de la réponse maximale. Ensuite, le processus de plasticité à long terme a été induit par application de 4 trains de 100 Hz espacés de 5 minutes. La réponse augmentée a été mesurée durant 4 heures. Pour chaque expérience, une voie de contrôle a également été mesurée, cette voie ne recevant pas de stimulation à haute fréquence.

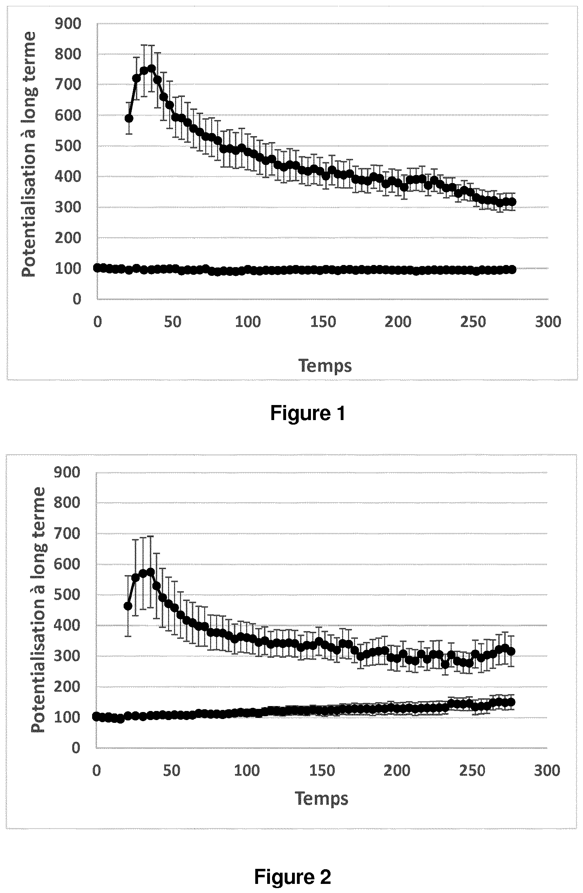

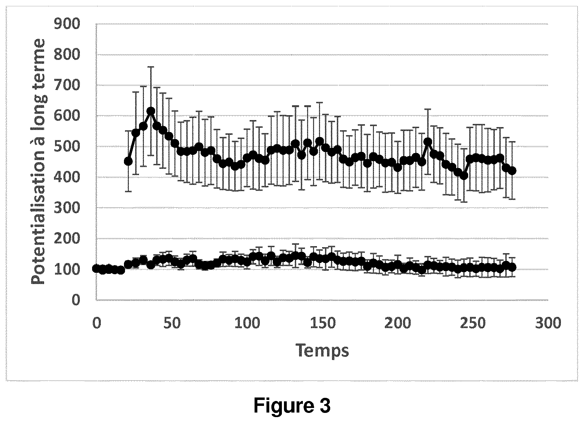

Les figures 1 à 3 illustrent les résultats obtenus pour chacun des Groupes. De ces figures, il peut être noté que :
- pour les rats du Groupe 1 (groupe contrôle), une voie de contrôle très stable est observée ainsi qu'une potentialisation à long terme supérieure à 300% (figure 1);
- pour les rats du Groupe 2 (rats carencés en magnésium), une voie de contrôle instable et augmentant au cours du temps est observée ainsi qu'une potentialisation à long terme inférieure à 300% (figure 2);
- pour les rats du Groupe 3 (rats carencés en magnésium mais supplémentés avec une composition selon l'invention comprenant du N-acétyl-taurinate de magnésium dihydraté comprenant deux molécules d'eau intrinsèques), une stabilisation de la ligne de base est observée ainsi qu'une potentialisation à long terme supérieure à 300% (figure 3).

Ces résultats permettent de mettre en avant, en particulier en comparant les résultats obtenus entre les Groupes 2 et 3, qu'une composition selon l'invention comprenant du N-acétyl-taurinate de magnésium dihydraté comprenant deux molécules d'eau intrinsèques assure une récupération/restauration de la plasticité synaptique.

La présente invention a été décrite en relation avec des modes de réalisations spécifiques, qui ont une valeur purement illustrative et ne doivent pas être considérés comme limitatifs. D'une manière générale, il apparaîtra évident pour l'homme du métier que la présente invention n'est pas limitée aux exemples illustrés et/ou décrits ci-dessus.

L'usage des verbes « comprendre », « inclure », « comporter », ou toute autre variante, ainsi que leurs conjugaisons, ne peut en aucune façon exclure la présence d'éléments autres que ceux mentionnés.

L'usage de l'article indéfini « un », « une », ou de l'article défini « le », « la » ou « l' », pour introduire un élément n'exclut pas la présence d'une pluralité de ces éléments.

## Revendications

1. Composé pour utilisation dans le traitement préventif et/ou curatif des maladies ou des troubles du système nerveux central **caractérisés par** un déclin de la plasticité neuronale, en particulier **caractérisés par** un déclin de la plasticité synaptique, par exemple dans le traitement des maladies ou des troubles du système nerveux central affectant les processus cognitifs tels que l'apprentissage, la perception, l'attention, le raisonnement, le langage et/ou la mémoire, dans le traitement des maladies du système nerveux central telles que les maladies neurodégénératives de type Parkinson, Alzheimer et/ou sclérose latérale amyotrophique, dans le traitement des maladies ou des troubles du système nerveux central responsables de troubles de l'humeur, d'hyperexcitabilité, d'hyperactivité et/ou de stress, dans le traitement d'affections neurologiques telle que l'épilepsie, ledit composé étant du N-acétyl-taurinate de magnésium dihydraté comprenant deux molécules d'eau intrinsèques.

2. Composé pour utilisation selon la revendication 1, ledit composé se présentant sous une forme administrable oralement, par exemple sous forme d'un comprimé, d'une capsule, d'une gélule, d'un cachet, d'une poudre soluble, d'une solution huileuse, d'un comprimé effervescent ou d'une gélule molle.

3. Composé pour utilisation selon la revendication 1 ou 2, ledit composé étant administré à raison de 1 à 2 capsules par jour chez l'adulte, l'adolescent ou l'enfant.

4. Composition pour utilisation dans le traitement préventif et/ou curatif des maladies ou des troubles du système nerveux central **caractérisés par** un déclin de la plasticité neuronale, en particulier **caractérisés par** un déclin de la plasticité synaptique, par exemple dans le traitement des maladies ou des troubles du système nerveux central affectant les processus cognitifs tels que l'apprentissage, la perception, l'attention, le raisonnement, le langage et/ou la mémoire, dans le traitement des maladies du système nerveux central telles que les maladies neurodégénératives de type Parkinson, Alzheimer et/ou sclérose latérale amyotrophique, dans le traitement des maladies ou des troubles du système nerveux central responsables de troubles de l'humeur, d'hyperexcitabilité, d'hyperactivité et/ou de stress, dans le traitement d'affections neurologiques telle que l'épilepsie, ladite composition comprenant au moins du N-acétyl-taurinate de magnésium dihydraté comprenant deux molécules d'eau intrinsèques.

5. Composition pour utilisation selon la revendication 4, dans laquelle ledit N-acétyl-taurinate de magnésium dihydraté comprenant deux molécules d'eau intrinsèques est présent à raison de 40 à 60% en poids par rapport au poids total de la composition.

6. Composition pour utilisation selon la revendication 4 ou 5, laquelle comprend en outre de la taurine à raison de 10 à 15% en poids par rapport au poids total de la composition.

7. Composition pour utilisation selon l'une quelconque des revendications 4 à 6, laquelle comprend en outre au moins un composé magnésique additionnel choisi dans le groupe constitué des sels magnésiques d'acides aminés, du citrate de magnésium, du gluconate de magnésium, du lactate de magnésium, du malate de magnésium, du taurate de magnésium, du bisglycinate de magnésium, du glycérophosphate de magnésium, du thréonate de magnésium, leurs dérivés et leurs mélanges.

8. Composition pour utilisation selon l'une quelconque des revendications 4 à 7, laquelle comprend en outre du magnésium à raison de 8 à 15% en poids par rapport au poids total de la composition.

9. Composition pour utilisation selon l'une quelconque des revendications 4 à 8, laquelle comprend en outre au moins un acide aminé, par exemple de la thréonine.

10. Composition pour utilisation selon la revendication 9, dans laquelle ledit au moins un acide aminé est présent à raison de 10 à 15% en poids par rapport au poids total de la composition.

11. Composition pour utilisation selon l'une quelconque des revendications 4 à 10, laquelle comprend en outre au moins une vitamine choisie dans le groupe constitué de la vitamine B1, B2, B6, et de leurs mélanges.

12. Composition pour utilisation selon la revendication 11, dans laquelle ladite au moins une vitamine est présente à raison de 0,05 à 0,15% en poids par rapport au poids total de la composition.

13. Composition pour utilisation selon l'une quelconque des revendications 4 à 12, comprenant en outre au moins un excipient pharmaceutiquement acceptable, de préférence quatre excipients pharmaceutiquement acceptables, choisi dans le groupe constitué du glycéryl béhénate, du stéarate de saccharose, de la cellulose microcristalline, du stéarate de magnésium, du dioxyde de silice, de la maltodextrine de pois et leurs mélanges.

14. Composition pour utilisation selon l'une quelconque des revendications 4 à 13, ladite composition se présentant sous la forme d'un médicament ou d'un complément alimentaire ou d'une boisson.

15. Composition pour utilisation selon l'une quelconque des revendications 4 à 14, ladite composition se présentant sous une forme administrable oralement, par exemple sous forme d'un comprimé, d'une capsule, d'une gélule, d'un cachet, d'une poudre soluble, d'une solution huileuse, d'un comprimé effervescent ou d'une gélule molle.

16. Composition pour utilisation selon l'une quelconque des revendications 4 à 15, ladite composition étant administrée à raison de 1 à 2 capsules par jour chez l'adulte, l'adolescent ou l'enfant.

## Patentansprüche

1. Verbindung zur Verwendung bei der präventiven und/oder heilenden Behandlung von Erkrankungen oder Störungen des zentralen Nervensystems, die durch einen Abfall der neuronalen Plastizität gekennzeichnet sind, insbesondere durch einen Abfall der synaptischen Plastizität gekennzeichnet sind, zum Beispiel bei der Behandlung von Erkrankungen oder Störungen des zentralen Nervensystems, die kognitive Prozesse wie Lernen, Wahrnehmung, Aufmerksamkeit, Denken, Sprache und/oder Gedächtnis beeinträchtigen, bei der Behandlung von Erkrankungen des zentralen Nervensystems wie neurodegenerativen Erkrankungen vom Typ Parkinson, Alzheimer und/oder amyotropher Lateralsklerose, bei der Behandlung von Erkrankungen oder Störungen des zentralen Nervensystems, die für Stimmungsschwankungen, Übererregbarkeit, Hyperaktivität und/oder Stress verantwortlich sind, bei der Behandlung von neurologischen Erkrankungen wie Epilepsie, wobei die Verbindung N-Acetyl-Magnesiumtaurinatdihydrat ist, das zwei intrinsische Wassermoleküle umfasst.

2. Verbindung zur Verwendung nach Anspruch 1, wobei die Verbindung in einer oral verabreichbaren Form vorliegt, zum Beispiel in Form einer Tablette, einer Kapsel, einer Gelatinekapsel, einer Pille, eines löslichen Pulvers, einer ölhaltigen Lösung, einer Brausetablette oder einer weichen Gelatinekapsel.

3. Verbindung zur Verwendung nach Anspruch 1 oder 2, wobei die Verbindung in einer Menge von 1 bis 2 Kapseln pro Tag an Erwachsene, Jugendliche oder Kinder verabreicht wird.

4. Zusammensetzung zur Verwendung bei der präventiven und/oder heilenden Behandlung von Erkrankungen oder Störungen des zentralen Nervensystems, die durch einen Abfall der neuronalen Plastizität gekennzeichnet sind, insbesondere durch einen Abfall der synaptischen Plastizität gekennzeichnet sind, zum Beispiel bei der Behandlung von Erkrankungen oder Störungen des zentralen Nervensystems, die kognitive Prozesse wie Lernen, Wahrnehmung, Aufmerksamkeit, Denken, Sprache und/oder Gedächtnis beeinträchtigen, bei der Behandlung von Erkrankungen des zentralen Nervensystems wie neurodegenerativen Erkrankungen vom Typ Parkinson, Alzheimer und/oder amyotropher Lateralsklerose, bei der Behandlung von Erkrankungen oder Störungen des zentralen Nervensystems, die für Stimmungsschwankungen, Übererregbarkeit, Hyperaktivität und/oder Stress verantwortlich sind, bei der Behandlung von neurologischen Erkrankungen wie Epilepsie, wobei die Zusammensetzung mindestens ein N-Acetyl-Magnesiumtaurinatdihydrat umfasst, das zwei intrinsische Wassermoleküle umfasst.

5. Zusammensetzung zur Verwendung nach Anspruch 4, wobei das N-Acetyl-Magnesiumtaurinatdihydrat, das zwei intrinsische Wassermoleküle umfasst, im Umfang von 40 bis 60 Gew.-% in Bezug auf das Gesamtgewicht der Zusammensetzung vorhanden ist.

6. Zusammensetzung zur Verwendung nach Anspruch 4 oder 5, die ferner Taurin im Umfang von 10 bis 15 Gew.-% in Bezug auf das Gesamtgewicht der Zusammensetzung umfasst.

7. Zusammensetzung zur Verwendung nach irgendeinem der Ansprüche 4 bis 6, die ferner mindestens eine zusätzliche Magnesiumverbindung umfasst, die aus der Gruppe ausgewählt ist, die aus Magnesiumsalzen von Aminosäuren, Magnesiumcitrat, Magnesiumglukonat, Magnesiumlaktat, Magnesiummalat, Magnesiumtaurat, Magnesiumbisglycinat, Magnesiumglycerophosphat, Magnesiumthreonat, deren Derivaten und deren Mischungen besteht.

8. Zusammensetzung zur Verwendung nach irgendeinem der Ansprüche 4 bis 7, die ferner Magnesium im Umfang von 8 bis 15 Gew.-% in Bezug auf das Gesamtgewicht der Zusammensetzung umfasst.

9. Zusammensetzung zur Verwendung nach irgendeinem der Ansprüche 4 bis 8, die ferner mindestens eine Aminosäure, zum Beispiel Threonin, umfasst.

10. Zusammensetzung zur Verwendung nach Anspruch 9, in welcher die mindestens eine Aminosäure im Umfang von 10 bis 15 Gew.-% in Bezug auf das Gesamtgewicht der Zusammensetzung vorhanden ist.

11. Zusammensetzung zur Verwendung nach irgendeinem der Ansprüche 4 bis 10, die ferner mindestens ein Vitamin umfasst, das aus der Gruppe ausgewählt ist, die aus Vitamin B1, B2, B6 und deren Mischungen besteht.

12. Zusammensetzung zur Verwendung nach Anspruch 11, in welcher das mindestens eine Vitamin im Umfang von 0,05 bis 0,15 Gew.-% in Bezug auf das Gesamtgewicht der Zusammensetzung vorhanden ist.

13. Zusammensetzung zur Verwendung nach irgendeinem der Ansprüche 4 bis 12, ferner umfassend mindestens einen pharmazeutisch akzeptablen Hilfsstoff, bevorzugt vier pharmazeutisch akzeptable Hilfsstoffe, die aus der Gruppe ausgewählt sind, die aus Glycerylbehenat, Saccharosestearat, mikrokristalliner Zellulose, Magnesiumstearat, Siliziumdioxid, Erbsenmaltodextrin und deren Mischungen besteht.

14. Zusammensetzung zur Verwendung nach irgendeinem der Ansprüche 4 bis 13, wobei die Zusammensetzung in Form eines Medikaments oder einer Nahrungsmittelergänzung oder eines Getränks vorliegt.

15. Zusammensetzung zur Verwendung nach irgendeinem der Ansprüche 4 bis 14, wobei die Zusammensetzung in einer oral verabreichbaren Form vorliegt, zum Beispiel in Form einer Tablette, einer Kapsel, einer Gelatinekapsel, einer Pille, eines löslichen Pulvers, einer ölhaltigen Lösung, einer Brausetablette oder einer weichen Gelatinekapsel.

16. Zusammensetzung zur Verwendung nach irgendeinem der Ansprüche 4 bis 15, wobei die Zusammensetzung in einer Menge von 1 bis 2 Kapseln pro Tag an Erwachsene, Jugendliche oder Kinder verabreicht wird.

## Claims

1. A compound for use in the preventive and/or curative treatment of diseases or disorders of the central nervous system **characterized by** a decline in neuronal plasticity, in particular **characterized by** a decline in the synaptic plasticity, for example in the treatment of diseases or disorders of the central nervous system affecting the cognitive processes such as learning, perception, attention, reasoning, language and/or memory, in the treatment of diseases of the central nervous system such as the neurodegenerative diseases of the Parkinson's, Alzheimer's and/or amyotrophic lateral sclerosis type, in the treatment of diseases or disorders of the central nervous system responsible for mood disorders, hyperexcitability, hyperactivity and/or stress, in the treatment of neurological disorders such as epilepsy, said compound being magnesium N-acetyl-taurinate dihydrate comprising two intrinsic water molecules.

2. The compound for use according to claim 1, said compound being in an orally administrable form, e.g., in the form of a tablet, a capsule, a soft gel, a pill, a soluble powder, an oily solution, an effervescent tablet or a soft gel capsule.

3. The compound for use according to claim 1 or 2, said compound being administered at a rate of 1 to 2 capsules per day in adults, adolescents or children.

4. A composition for use in the preventive and/or curative treatment of diseases or disorders of the central nervous system **characterized by** a decline in neuronal plasticity, in particular, **characterized by** a decline in synaptic plasticity, for example in the treatment of diseases or disorders of the central nervous system affecting the cognitive processes such as learning, perception, attention, reasoning, language and/or memory, in the treatment of diseases of the central nervous system such as neurodegenerative diseases of the Parkinson's, Alzheimer's and/or amyotrophic lateral sclerosis type, in the treatment of diseases or disorders of the central nervous system causing nervous system responsible for mood disorders, hyperexcitability, hyperactivity and/or stress, in the treatment of neurological disorders such as epilepsy, said composition comprising at least magnesium N-acetyl-taurinate dihydrate comprising two intrinsic water molecules.

5. The composition for use according to claim 4, wherein said magnesium N-acetyl-taurinate dihydrate comprising two intrinsic water molecules is present in an amount of from 40 to 60% by weight based on the total weight of the composition.

6. The composition for use according to claim 4 or 5, which further comprises taurine in an amount of from 10 to 15% by weight based on the total weight of the composition.

7. The composition for use according to any one of claims 4 to 6, which further comprises at least one additional magnesium compound selected from the group consisting of magnesium salts of amino acids, magnesium citrate, magnesium gluconate, magnesium lactate, magnesium malate, magnesium taurate, magnesium bisglycinate, magnesium glycerophosphate, magnesium threonate, derivatives thereof and mixtures thereof.

8. The composition for use according to any one of claims 4 to 7, which further comprises magnesium in an amount of from 8 to 15% by weight based on the total weight of the composition.

9. The composition for use according to any one of claims 4 to 8, which further comprises at least one amino acid, for example threonine.

10. The composition for use according to claim 9, wherein said at least one amino acid is present in an amount of from 10 to 15% by weight based on the total weight of the composition.

11. The composition for use according to any one of claims 4 to 10, which further comprises at least one vitamin selected from the group consisting of vitamin B1, B2, B6, and mixtures thereof.

12. The composition for use according to claim 11, wherein said at least one vitamin is present in an amount of 0.05 to 0.15% by weight based on the total weight of the composition.

13. The composition for use according to any one of claims 4 to 12, further comprising at least one pharmaceutically acceptable excipient, preferably four pharmaceutically acceptable excipients, selected from the group consisting of glyceryl behenate, sucrose stearate, microcrystalline cellulose, magnesium stearate, silica dioxide, pea maltodextrin and mixtures thereof.

14. The composition for use according to any one of claims 4 to 13, said composition being in the form of a medicament or a dietary supplement or a beverage.

15. The composition for use according to any one of claims 4 to 14, said composition being in an orally administrable form, e.g., in the form of a tablet, a capsule, a soft gel, a pill, a soluble powder, an oily solution, an effervescent tablet or a soft gel capsule.

16. The composition for use according to any one of claims 4 to 15, said composition being administered at a rate of 1 to 2 capsules per day in adults, adolescents or children.
